# EUROPEAN PATENT APPLICATION

(11) **EP 4 640 163 A2**
(43) Date of publication of application: **29.10.2025**
(21) Application number: 25201226.5
(22) Date of filing: 30.12.2021
(51) Int. Cl.: A61B 17/00

(54) **APPARATUS AND METHOD FOR IMPLANTING AN ARTERIOVENOUS GRAFT**

(30) Priority: 26.03.2021 US 202163166794 P; 26.03.2021 US 202163166790 P
(62) Divisional of application: 21848111.7
(71) Applicant: Innavasc Medical, Inc., Durham, North Carolina 27705 (US)
(72) Inventor: GAGE, Shawn M., Raleigh, North Carolina, 27613 (US); LUCAS, John, Greenwood, Mississippi, 38930 (US); NICHOLS, Craig, Durham, North Carolina, 27701 (US); KNIGHT, Joseph, Durham, North Carolina, 27705 (US); CHIOSONNE, Juan, Raleigh, North Carolina, 27603 (US); LAWSON, Peter, Durham, North Carolina, 27707 (US); BUSCH, Samantha, Indian Land, South Carolina, 29707 (US); WALSH, Brian, Charlotte, North Carolina, 28273 (US)
(74) Representative: HGF

(57) **Abstract**

An apparatus for delivery of a vascular arteriovenous graft comprises a tubular sleeve defining an interior lumen adapted for being slidably positioned over a tunneling instrument. Once the tunneling instrument has been advanced to a desired subcutaneous anatomical location the shaft is removed while the sleeve remains in the tissue. The sleeve has a linear slit extending from a proximal end to a point intermediate the length of the sleeve. The lumen receives the distal end of the graft and at least a portion of a cannulation chamber such that the end of the sleeve progressively expands along the slit to provide an enlarged diameter for accommodating the cannulation chamber. Longitudinal force to the sleeve during removal of the sleeve from the tissue secures the graft in the sleeve by radial compression of the sleeve for pulling the graft and the sleeve through the tunnel and deploying the vascular graft.

## Description

### Cross-References to Related Applications

This application claims the benefit of U.S. Provisional Application No. 63/166,794 and U.S. Provisional Application No. 63/166,790, both filed March 26, 2021, the contents of both of which are incorporated herein in their entirety.

### Background

An apparatus and method are described for implanting an arteriovenous graft and, more particularly, an apparatus and method for using a tunneling instrument in forming a subcutaneous anatomical tunnel for implanting the arteriovenous graft, including use of a removable sleeve enclosing the tunneling instrument for delivery of the graft.

A vascular arteriovenous graft is a tubular device that is suitable for implantation in the body to redirect flow of blood between blood vessels. Surgical implantation of the arteriovenous graft requires placement of the graft within subcutaneous tissue. An initial step in the implantation procedure is creation of a subcutaneous anatomic pathway, or "tunnel", for passage of the arteriovenous graft, which is commonly called a graft tunnel, between anastomotic sites. This is a required surgical step in peripheral vascular procedures for all peripheral, vascular access and extra-anatomical graft locations. The arteriovenous graft is positioned in the tunnel within the bodily tissue for fixation of the graft to an existing peripheral vessel to form a bypass around the vessel, or a portion thereof, or connection of an artery and vein to form an arteriovenous shunt. The vascular graft may also connect an artery to an artery.

A conventional tunneling device includes an elongated rigid rod having a handle on a proximal end and a bullet-shaped tip at a distal end. The rod may vary in size and shape and may have a straight shaft, a curved shaft or a semicircular shaft, which allows for a variety of graft placement positions and locations. In the tunneling procedure, a first proximal incision and a second distal incision are made at a chosen area of anastomosis. The tip at the distal end of the tunneling device is inserted into the proximal incision. The tip of the tunneling device is then forcefully passed through the subcutaneous tissue creating a path between the incisions by blunt dissection until the tip protrudes from the distal incision. Once the tip is exposed, a proximal end of the arteriovenous graft is tied onto the distal end or the tip of the tunneling device with sterile suture thread. The tunneling instrument and attached arteriovenous graft are then pulled proximally along the path through the recently dissected graft tunnel until the proximal end of the arteriovenous graft extends from the proximal incision. When the arteriovenous graft is appropriately positioned, the graft is cut free from the distal end of the tunneling instrument, removing a portion of the graft. An anastomosis is formed between the ends of the graft and the blood vessels around the area of vasculature to be bypassed and the incisions are closed.

The step of pulling the tunneling instrument and attached arteriovenous graft through the graft tunnel can require significant force. The force required depends on a number of factors, including the relative sizes of the graft tunnel and the graft and the material of the graft. Conventional delivery systems for arteriovenous grafts and other implantable devices are sometimes covered by a retaining sleeve that reduces the friction of passage through the subcutaneous tissue. Following implantation, the sleeve is removed by pulling or rolling back over the device to retract the sleeve. While rolling a sleeve during retraction reduces the necessary pulling force as compared to withdrawing the sleeve by sliding the sleeve over the device, there still can be significant force necessary to retract a sleeve following implantation of an arteriovenous graft.

For the foregoing reasons, there is a need for an apparatus and method for implanting an arteriovenous graft for minimizing force necessary to deliver the graft. The new apparatus in the form of a sleeve should help define and support the length of the anatomical tunnel formed by the tunneling device. The sleeve should be extractable in a reliable manner with a low pulling force for minimizing problems associated with excessive axial forces on the sleeve during retraction. The new apparatus and method should also be capable of implanting any type of vascular graft including, but not limited to, ePTFE and a natural tissue graft.

### Summary

An apparatus is provided for subcutaneous delivery in tissue of a patient of a vascular arteriovenous graft, including a cannulation chamber and having a proximal end and a distal end and a length extending along a longitudinal axis between the proximal end and the distal end. The apparatus using a rigid tunneling instrument including an elongated shaft having a handle at a proximal end of the shaft and a removable tip at a distal end of the shaft for creating a pathway in the subcutaneous tissue. The subcutaneous delivery apparatus comprising an elongated tubular sleeve having a length and defining an interior lumen, the sleeve adapted for being slidably positioned over at least a portion of the shaft of the tunneling instrument between the handle and the tip. Once the tunneling instrument has been advanced to a desired subcutaneous anatomical location the shaft is selectively removed from the sleeve while the sleeve remains positioned at the desired anatomical location. The sleeve has a linear slit extending along the length of the sleeve from the proximal end to a point intermediate the length of the sleeve. The lumen defined at the proximal end of the sleeve is adapted to receive the distal end of the graft and at least a portion of the cannulation chamber such that the proximal end portion of the sleeve progressively expands along the slit to provide an enlarged diameter for accommodating the cannulation chamber. Application of a longitudinal force to the sleeve from the distal end of the sleeve is effective to cause the sleeve to move in a distal direction during removal of the sleeve from the tissue such that the graft is fixed in the sleeve by radial compression of the sleeve for pulling the graft and the sleeve through the tunnel deploying the vascular graft.

In one aspect, the sleeve is adapted to receive substantially the entire length of the shaft between the handle and the tip. In another aspect, the sleeve is uniformly tapered from the proximal end to the distal such that distal end has a reduced diameter from the proximal end.

In one embodiment, the sleeve comprises an enlarged diameter portion adjacent the proximal end and adapted for receiving the cannulation chamber.

An apparatus is also provided for subcutaneous implantation in tissue of a patient of a vascular arteriovenous graft including a cannulation chamber and having a proximal end and a distal end and a length extending along a longitudinal axis between the proximal end and the distal end. The subcutaneous implantation apparatus comprises a rigid tunneling instrument for creating a pathway in the subcutaneous tissue. The tunneling instrument includes an elongated shaft having a handle at a proximal end of the shaft and a removable tip at a distal end of the shaft. An elongated tubular sleeve has a length and defines an interior lumen. A linear slit extends along the length of the sleeve from the proximal end to a point intermediate the length of the sleeve. The sleeve is configured for being slidably positioned over at least a portion of the shaft of the tunneling instrument between the handle and the tip. Once the tunneling instrument has been advanced to a desired subcutaneous anatomical location the shaft is selectively removed from the sleeve while the sleeve remains positioned at the anatomical location. The lumen is adapted to receive the distal end of the graft and at least a portion of the cannulation chamber via the proximal end of the sleeve such that the proximal end portion of the sleeve progressively expands along the slit to provide an enlarged diameter for accommodating the cannulation chamber. Application of a longitudinal force to the sleeve from the distal end of the sleeve is effective to cause the sleeve to move in a distal direction for removal of the sleeve from the tissue such that the arteriovenous graft is fixed in the sleeve by radial compression of the tissue around the sleeve. Pulling the arteriovenous graft and the sleeve through the tunnel deploying the vascular graft.

In one aspect, the sleeve is adapted to receive substantially the entire length of the shaft between the handle and the tip. In another aspect, the sleeve is uniformly tapered from the proximal end to the distal such that distal end has a reduced diameter from the proximal end.

In one embodiment, the sleeve comprises an enlarged diameter portion adj acent the proximal end and adapted for receiving the cannulation chamber.

A feature of the sleeve comprises a wall at the proximal end, the wall having an axial opening for receiving the tip of the tunneling instrument for connection of the sleeve to the tunneling instrument. A clip including a pin wherein the shaft has a hole adjacent the tip defining an axial passage for receiving the pin.

A system is provided for subcutaneous delivery of a medical device in a patient. The subcutaneous delivery system comprises a tunneling instrument including a shaft having a proximal end and a distal end, a vascular graft having a length, a distal end, a proximal end, an outer surface and a longitudinal axis, and a sleeve having a length and being positioned over a substantial portion of the outer surface of the shaft. The sleeve is configured to exert radial pressure on the cannulation chamber upon application of a longitudinal force to the sleeve in a distal direction for implantation of the vascular graft to cause the sleeve to move in a distal direction during removal of the sleeve.

In one aspect, the sleeve is adapted to receive substantially the entire length of the shaft between the handle and the tip. In another aspect, the sleeve is uniformly tapered from the proximal end to the distal such that distal end has a reduced diameter from the proximal end.
In one embodiment, the sleeve comprises an enlarged diameter portion adj acent the proximal end and adapted for receiving the cannulation chamber.

A feature of the sleeve comprises a wall at the proximal end, the wall having an axial opening for receiving the tip of the tunneling instrument for connection of the sleeve to the tunneling instrument.

In another embodiment, the subcutaneous delivery system may further comprise a clip including a pin wherein the shaft has a hole adjacent the tip defining an axial passage for receiving the pin for connecting the shaft to the sleeve for applying a pulling force in a proximal direction.

A method for subcutaneously implanting an arteriovenous graft in tissue of a subject is also provided. The arteriovenous graft includes a cannulation chamber, a proximal end and a distal end and a length extending along a longitudinal axis between the proximal end and the distal end. The implanting method comprises the steps of incising tissue of the subject in a first proximal location and second distal location spaced from the first proximal location. A rigid tunneling instrument is provided, including an elongated shaft having a handle at a proximal end of the shaft and a removable tip at a distal end of the shaft. The tip is removed and a tubular sleeve disposed over at least a portion of the shaft between the tip and handle, the sleeve having a slit extending along the length of the sleeve from the proximal end to a point intermediate the length of the sleeve. The tip is secured to the distal end of the shaft and inserted into the first incision and the user subcutaneously advances the instrument and the sleeve through the tissue along a path until the tip exits the second incision such that the distal end of the sleeve extends from the second incision and the proximal end of the sleeve extends from the first incision. The tip is removed before pulling the tunneling instrument proximally by the handle for removal of the shaft from the sleeve in the tissue. The distal end of the graft is inserted into the proximal end of the sleeve until the cannulation chamber at least partially enters the sleeve causing the sleeve to expand along the slit to accommodate at least a portion of the chamber. The method includes the step of pulling the sleeve distally for extracting the sleeve from the tissue while the proximal end of the sleeve is compressed by the dissected tissue defining the tunnel forcing the sleeve against the chamber for pulling the graft through the tissue with the sleeve.

In one aspect, the method may further comprise the step of pulling the proximal end of the arteriovenous graft proximally for drawing the cannulation chamber of the arteriovenous graft back into the tissue.

### Brief Description Of The Drawings

For a more complete understanding of the apparatus and method for use in forming a subcutaneous anatomical tunnel, reference should now be had to the embodiments shown in the accompanying drawings and described below. In the drawings:
FIG. 1 is a perspective view from a distal end of a tapered sleeve for use with a tunneling instrument for subcutaneously deploying an arteriovenous graft.
FIG. 2 is a perspective view from a proximal end of the sleeve as shown in FIG. 1.
FIG. 3 is a proximal end view of the sleeve as shown in FIG. 1.
FIG. 4 is a distal end view of the sleeve as shown in FIG. 1.
FIG. 5 is a top plan view of the sleeve as shown in FIG. 1.
FIG. 6 is a bottom plan view of the sleeve as shown in FIG. 1.
FIG. 7 is an exploded perspective view of an apparatus for use in forming a subcutaneous anatomical tunnel and delivering an arteriovenous graft.
FIG. 8 is a perspective view of the tunnel forming and delivery apparatus as shown in FIG. 7 in a condition for tunneling with the sleeve on the tunneling instrument.
FIG. 9 is a close-up perspective view of a shaft of the tunneling instrument as shown in FIG. 7 being inserted into the sleeve as shown in FIG. 7.
FIG. 10 is a perspective view showing the tunneling instrument covered by the sleeve as shown in FIG. 8 and deployed in a portion of tissue and having ends extending from spaced incisions.
FIG. 11 is a perspective view as shown in FIG. 10 with the tunneling instrument removed and showing the sleeve in the tissue.
FIG. 12 is a perspective view as shown in FIG. 11 showing an arteriovenous graft including a cannulation chamber loaded into a proximal end of the sleeve.
FIG. 13 is a perspective view as shown in FIG. 12 showing the proximal end of the sleeve exiting the tissue and the cannulation chamber of the arteriovenous graft partially extending from the tissue.
FIG. 14 is a perspective view as shown in FIG. 13 with only the arteriovenous graft remaining in the tissue with both ends extending from the tissue at each incision.
FIG. 15 is a perspective view in cross-section of a proximal end of another embodiment of a sleeve for use with a tunneling instrument.
FIG. 16 is a perspective views in cross-section of a proximal end of a third embodiment of a sleeve for use with a tunneling instrument.
FIGs. 17A and 17B are perspective views of a fourth embodiment of a sleeve for use with a tunneling instrument.
FIG. 18 is a perspective view of a mechanical fastening element for connecting the sleeve to the tunneling instrument as shown in FIG. 1.
FIG. 19 is a perspective view of a fifth embodiment of a sleeve for use with a tunneling instrument for delivering an arteriovenous graft having cannulation chamber.
FIGs. 20A and 20B are perspective views of a sixth embodiment of the sleeve and tunneling instrument for delivery of an arteriovenous graft.

### Description

Certain terminology is used herein for convenience only and is not to be taken as a limiting. For example, words such as "upper," "lower," "left," "right," "horizontal," "vertical," "upward," "downward," "top" and "bottom" merely describe the configurations shown in the FIGs. Indeed, the components may be oriented in any direction and the terminology, therefore, should be understood as encompassing such variations unless specified otherwise. The words "interior" and "exterior" refer to directions toward and away from, respectively, the geometric center of the core and designated parts thereof. The terminology includes the words specifically mentioned above, derivatives thereof and words of similar import.

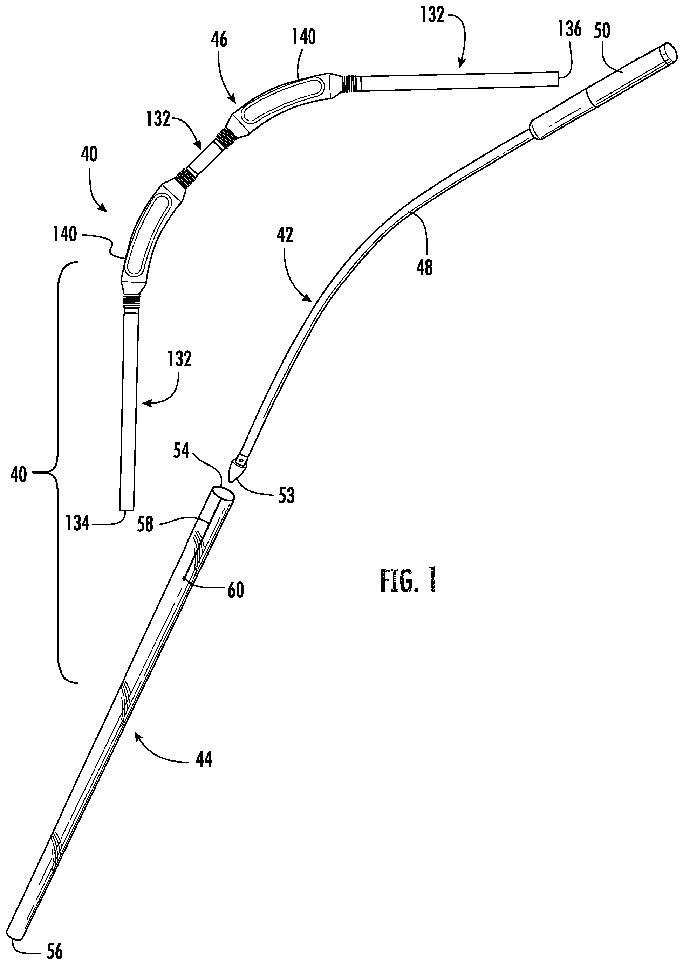

Referring now to the drawings, wherein like reference numerals designate corresponding or similar elements throughout the several views, an embodiment of an apparatus for use in forming a subcutaneous anatomical tunnel for implantation of a vascular arteriovenous graft in a patient is shown in FIGs. 1 and generally designated at 40. The tunnel forming and implanting apparatus 40 comprises a tunneling instrument 42, also referred to herein as a "tunneling device", a tubular sleeve 44 and a vascular arteriovenous graft 46. The tunneling instrument 42 includes an elongated rigid shaft 48, a proximal handle 50 at one end of the shaft and a removable distal tip 52 at the other end of the shaft. The tip 52 is generally bullet-shaped, or otherwise has an elliptical or circular shape, increasing in diameter from a pointed distal end. During the tunneling procedure, the shaft 48 and the tip 52 are advanced through the subcutaneous tissue by exerting axial force in the distal direction on the handle 50, with the tip facilitating the blunt dissection of tissue inherent in the tunneling procedure. The shaft 50 and tip 52 may be constructed of stainless steel, but one of ordinary skill in the art will recognize that other materials may be suitable. One such example of an alternative material is a plastic, such as a hard plastic. A suitable tunneling instrument is a Kelly-Wick Tunneler available from C.R. Bard, Inc. of Moncks Corner, South Carolina.

An arteriovenous graft 46 suitable for use in this apparatus and method is described in U.S. Patent No. 9,585,998, the contents of which are hereby incorporated by reference in their entirety. The arteriovenous graft 46 comprises a conduit 132 having a first end portion 134 and a second end portion 136. The first end portion 134 is configured to connect to a first blood vessel of a subject, such as an artery, at an end thereof. The second end portion 136 is configured to connect to a second blood vessel of the subject, such as a vein, at an end thereof. In this regard, blood flows through the conduit 132 from the first end portion 134 to the second end portion 136. At least one cannulation chamber 140 is positioned between the first end portion 134 and the second end portion 136 of the conduit 132. The conduit 132 extends through the chamber 140. The cannulation chamber 140 has an open anterior portion defining a cannulation port configured to receive a dialysis needle therethrough. It is understood that the tunnel forming apparatus is also capable of use with any vascular graft, as well as a natural tissue graft or fistula.

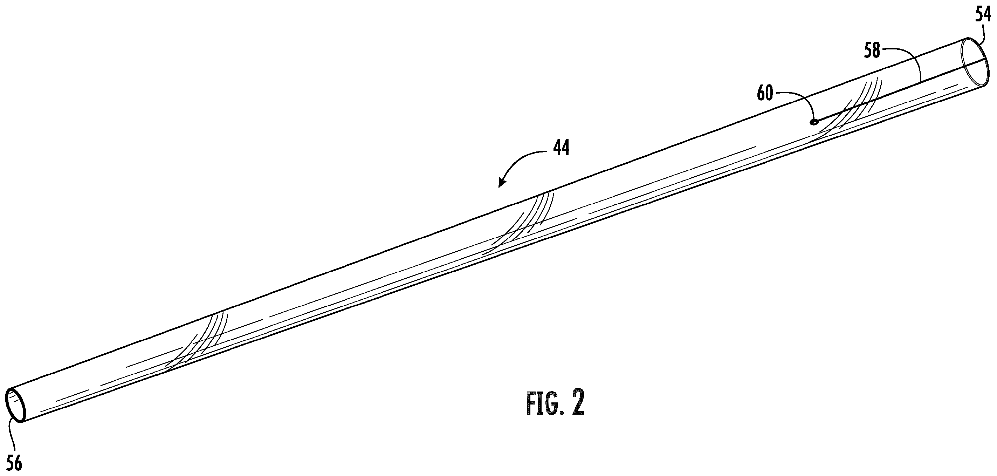

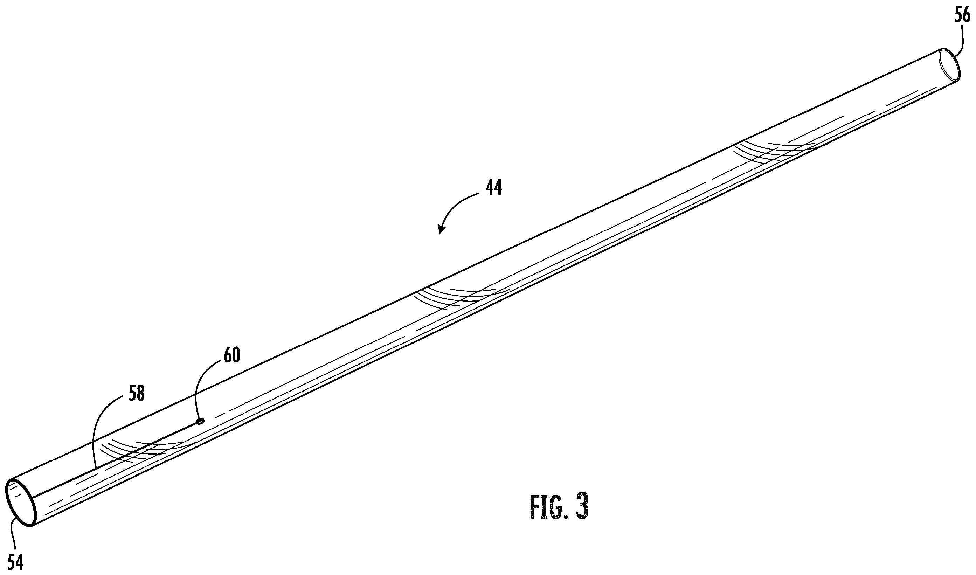

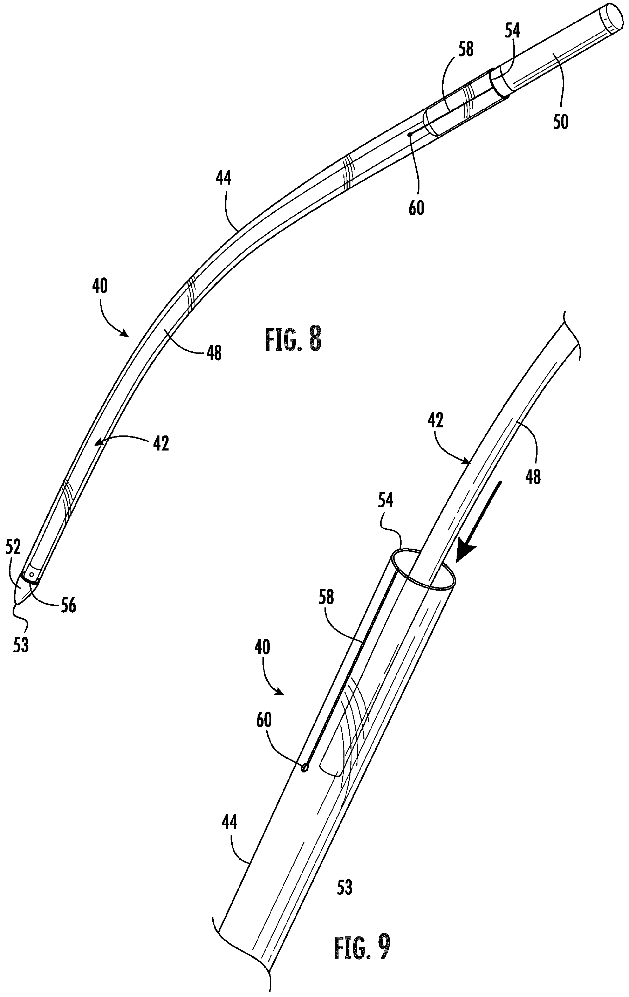

Referring to FIGs. 2-7, the tubular sleeve 44 comprises a hollow tube that is open at both a proximal end 54 and a distal end 56. In one embodiment, the sleeve 44 tapers in diameter from the proximal end 54 to the distal end 56. The diameter of the sleeve 44 at the distal end 56 is less than the largest of the tip 52. As shown in FIG. 8, the sleeve 44 proximally engages the tip 52 and extends to the handle 50 of the tunneling instrument 42. The sleeve 44 is dimensioned, in diameter and in length, to be slidably positioned over and enclose at least a portion of the shaft 48 of the tunneling instrument 42 during the tunneling procedure. Following tunneling of the tissue, the tip is removed 52 and the shaft 48 is extracted from the sleeve 44 upon application of a pulling force on the handle 50 along the longitudinal axis of the shaft 48 in the proximal direction. The sleeve 44 remains in the tissue and provides for insertion of the graft 46 through the tunneled tissue path due to the unrestricted and flexible nature of the surface of the sleeve 44.

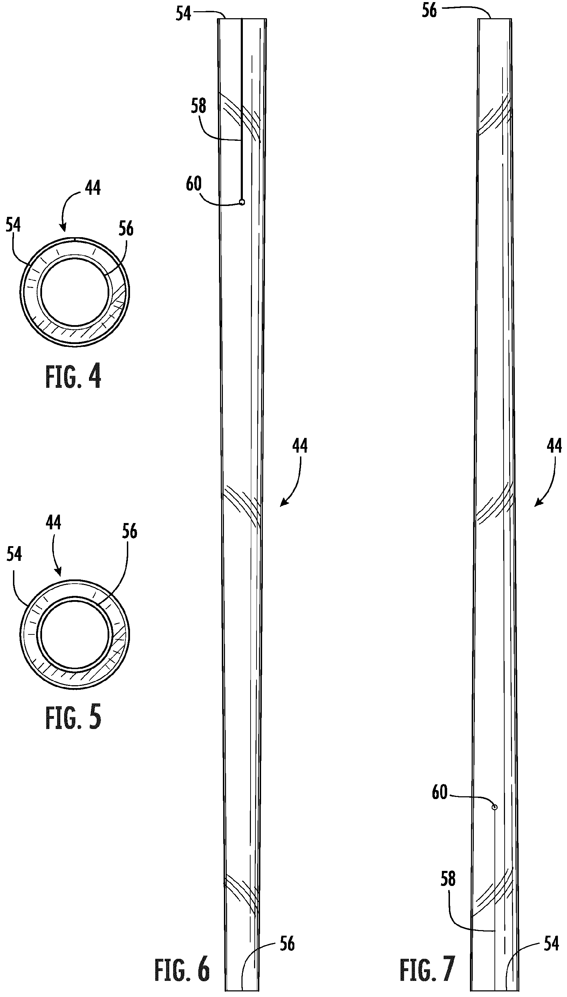

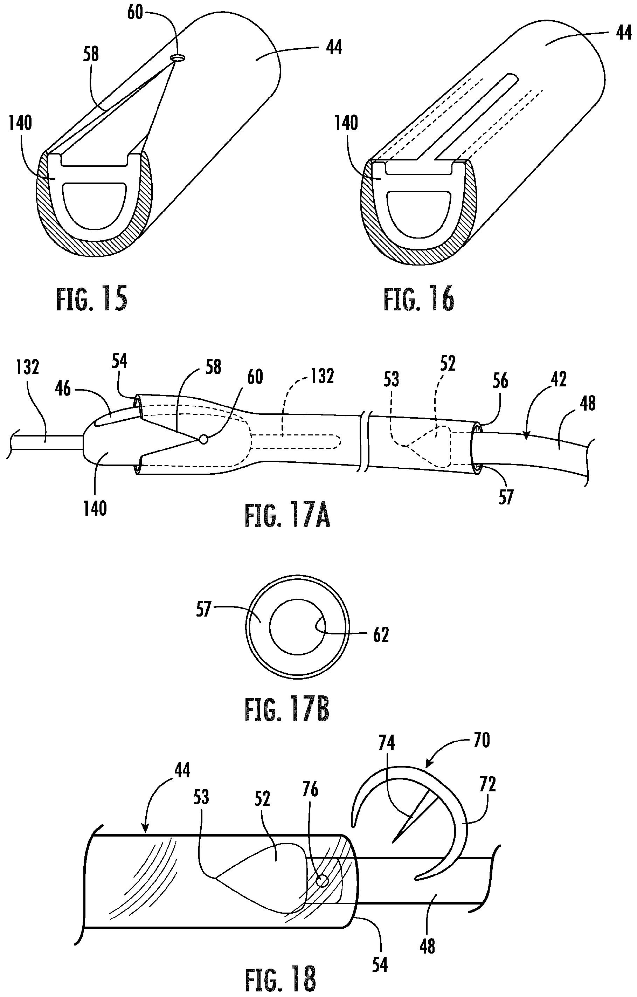

As best seen in FIGs. 6 and 7, the sleeve 44 has a linear slit 58 oriented along the longitudinal axis of the sleeve extending from the open proximal end 54 to a location along the length of the sleeve 44. The slit 58 may be generally oriented linearly or helically (not shown) along the length of the sleeve 44. The slit 58 allows the sleeve 44 to expand at the slit as an arteriovenous graft is inserted into the proximal end 54 of the sleeve. In the embodiment shown, the sleeve 44 has a hole 60 at the end of the slit 58 to prevent the sleeve from tearing from the slit 58 as the sleeve is expanded. The slit 58 can vary in length and shape. A shorter slit allows for receiving only a smaller area of the cannulation chamber 140 which may reduce surface damage. A longer slit 58 allows for receiving more of the cannulation chamber 140 for better clamping of the chamber 140 during delivery to reduce risk of the sleeve from releasing before the graft 46 is completely in the anatomical tunnel. Slit 58 openings can also have different shapes. FIG. 15 shows material removal from the proximal end 54 of the sleeve 44 for forming a triangular opening. Similarly, FIG. 16 shows a slit 58 forming a rectilinear opening for receiving the cannulation chamber 140. Multiple slits (not shown) may also be used. Multiple slits allow for larger cannulation chambers 140 to be inserted into and clamped by the sleeve 44. It is understood that a slit 58 in the end of the sleeve 44 is not necessary when the sleeve is used for deployment of conventional and biologic grafts. In this application there is no need for the slit since lumen is larger than the graft diameter. The graft may be simply slid into the sleeve 44 in the tissue, and the sleeve 44 is pulled out of the tissue leaving the graft behind.

The sleeve 44 can be constructed from any smooth, flexible and compressive biocompatible material. Suitable material can be porous, non-porous, permeable, or impermeable. The sleeve material does not excessively flex, so that the sleeve 44 can absorb the tensile force imparted during tunneling and deployment of the graft 46. Examples of such materials include, but are not limited to, silk, silicone, fluoropolymers such as expanded polytetrafluoroethylene (ePTFE), high density polyethylene (HDPE), and other polymers such as polyesters and polyimides. A suitable material is available from Colorite of Ridgefiled, New Jersey. Various desired configurations may be achieved by varying sleeve materials and characteristics, such as thickness and width. The sleeve 44 may be extruded. It is understood that the sleeve 44 may have a sufficiently low coefficient of friction that the sleeve can be removed from the anatomical tunnel by an axial pulling force on the sleeve.

In another embodiment, the sleeve 44 has a "double walled" construction. The double walled sleeve is formed from a double layer of thin flexible, compressible material closed at both ends. An inner portion, or first wall, of the sleeve 44 has a predetermined durometer and extends from a position at the proximal end 54 of the sleeve 44 radially over and axially along the sleeve to the distal end 56 of the sleeve. The outer portion, or second wall, of the sleeve extends radially over and axially along the inner portion from the proximal end 54 to the distal end 56 of the sleeve 44. The durometer of the inner portion of the double-walled sleeve 44 is lower than the durometer of the outer portion providing for a harder outer shell for the sleeve and a softer inner portion surrounding the graft 46 to prevent damage during deployment.

The smooth outer surface of the material of the sleeve may allow the sleeve 44 to have a low coefficient of friction. The result is easier insertion of the tunneling instrument 42 and the sleeve 44 through the tissue with less trauma, less friction, less blunt dissection and less drag during placement. Due to the smoothness and low profile of the sleeve 44 relative to the shaft 48, the sleeve does not substantially increase the outside diameter of the tunneling instrument 42.

The sleeve 44 may optionally be coated on an outside surface with a lubricious substance to provide a low coefficient of friction, aiding insertion and movement of the sleeve 44 and the associated tunneling instrument 42 through tissue while minimizing tissue drag and trauma during insertion or during sleeve removal after implantation. This will minimize tissue drag and tissue trauma during insertion of the tunneling instrument 42 and the sleeve 44 or removal of the sleeve 44 during implantation of the graft 46. It is understood that a wide variety of coatings are available, including therapeutic agents for delivery of therapeutic materials. Solid lubricants (i.e. graphite, waxes, silicone), fluid lubricants (i.e. hydrocarbon oils, silicone oils), gels (i.e. hydrogel) or any other biocompatible material known in the art may be used. In one embodiment, the sleeve 44 can be coated or wetted immediately before use. In another embodiment, the Applicant contemplates a kit comprising the sleeve 44 and a wetting agent for wetting the sleeve. In another embodiment, the kit comprises a sleeve 44, an arteriovenous graft 46 and a wetting agent for wetting the sleeve.

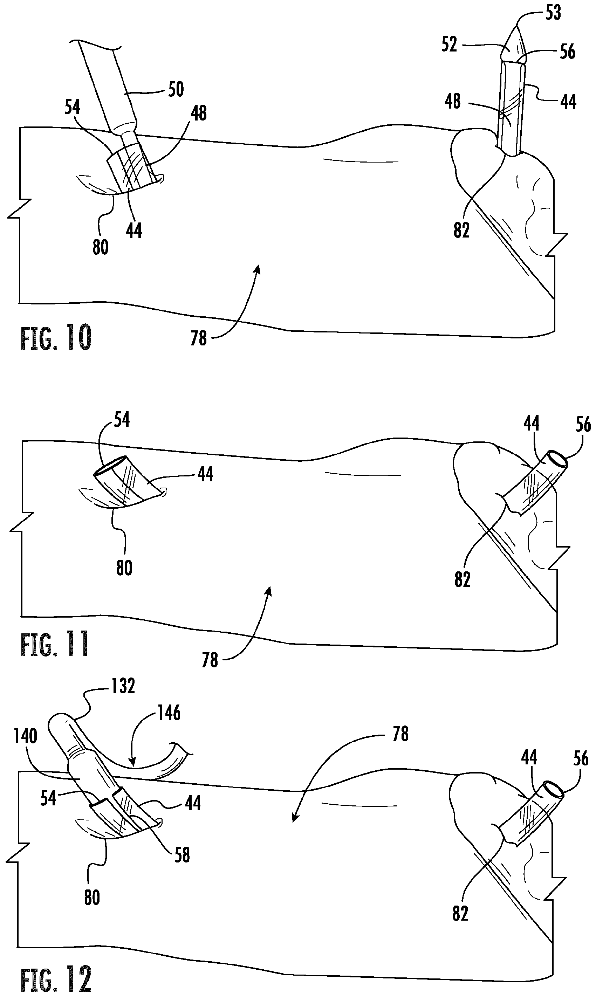

In use, a first proximal incision 80 and a second distal incision 82 spaced from the first incision are first made through the skin 78 of a patient into underlying subcutaneous tissue. The tip 52 at the distal end 53 of the tunneling instrument 42 with the sleeve 44 on the shaft 48 is inserted into the first incision 80 and then forced horizontally through the subcutaneous tissue along a path until the tip exits the second incision 82 (FIG. 10). The tip 52 is then removed by the surgeon and the tunneling instrument 42 pulled proximally by the handle 50 from the first incision 80 for removal of the shaft 48 from the tissue. The sleeve 44 is left within the tissue of the body. As shown in FIG. 11, the ends 54, 56 of the sleeve 44 extend proximally and distally from the first and second incisions 80, 82 externally of the skin 78.

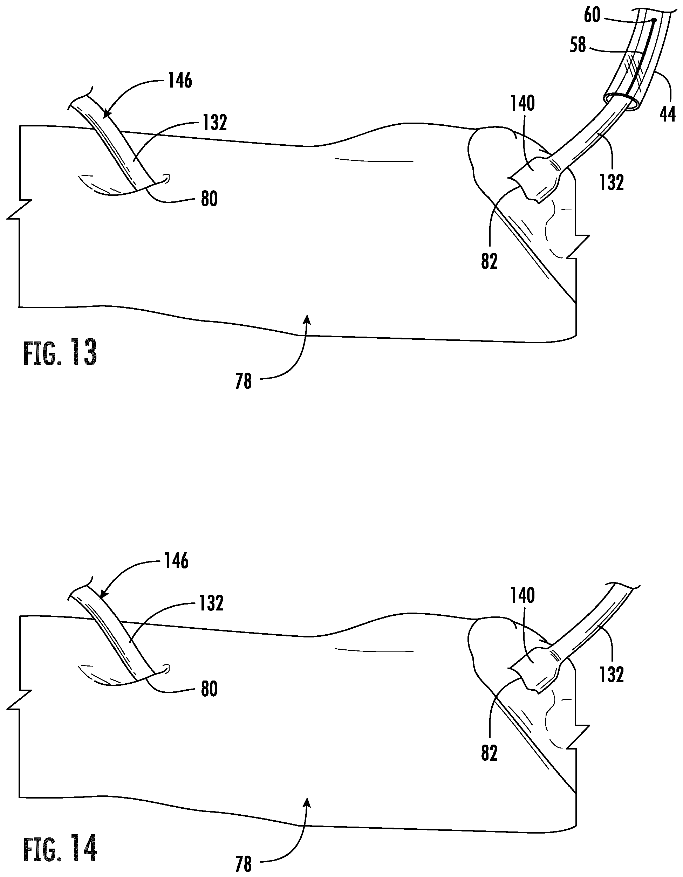

The distal end portion 136 of the graft 46 is inserted into the proximal end 54 of the sleeve 44 until the cannulation chamber 140 at least partially enters the sleeve 44 (FIG. 12). The sleeve 44 allows the surgeon to easily push the vascular graft 46 into the internal lumen defined by the sleeve, which is oversized in comparison to the outside diameter of the conduit 132 of the vascular graft 46. However, the sleeve 44 is configured such that the outer diameter of the cannulation chamber 140 is larger than the inner diameter of the sleeve. The cannulation chamber 140 thus causes the sleeve 44 to expand along the slit 58 to accommodate at least a portion of the chamber (FIG. 12). The distal end of the sleeve 44 extends sufficiently externally from the distal incision 82 so that it can be pulled upon for extracting the sleeve from the tissue. After placement of the distal portion of the graft 46 in the sleeve 44, the sleeve 44 is pulled and the proximal end 54 of the sleeve 44 is compressed by the dissected tissue defining the tunnel thereby forcing the sleeve against the cannulation chamber 140. The sleeve 44 is drawn axially distally until the sleeve 44 is extracted from the tissue while pulling the arteriovenous graft 46 into the tissue 78. The pulling force extracts the sleeve 44 from the distal incision 82 and moves the arteriovenous graft 46 progressively distally into the tissue 78 through the first incision 80. Without the tissue compressing the sleeve 44 against the cannulation chamber, the proximal end 54 of the sleeve 44 expands and releases the arteriovenous graft 46 when the proximal end 54 of the sleeve clears the distal incision 82. The arteriovenous graft 46 is left in the tissue extending from the incision (FIG. 14). The pulling force is depicted by arrows. The proximal end 54 of the arteriovenous graft 46 is then pulled proximally for drawing the cannulation chamber 140 of the arteriovenous graft 46 back into the tissue 78.

In another embodiment of the method of delivering an arteriovenous graft 46, the cannulation chamber 140 is held in the desired anatomical position through the skin 78 while the sleeve 44 can be drawn axially distally relative to the arteriovenous graft 46 and extracted from the subcutaneous tissue path from the second incision 82. Specifically, when the arteriovenous graft 46 is subcutaneously located as desired, the surgeon holds the graft 46 in place by pushing on the graft across the skin. Continued pulling extracts the sleeve 44 from over the arteriovenous graft 46 by the pulling force provided. The graft 46 remains within the anatomic subcutaneous tunnel. The surgeon now forms anastomoses at each conduit end 134, 136 of the graft 10 by suturing the ends of the graft to a blood vessel at the desired locations.

In another embodiment, the proximal end 54 of the sleeve 44 may be mechanically secured to the distal end 53 of the tunneling instrument 42 adjacent the tip 52 by mechanical or interference fit, mechanical structures, heat bonding or by a biocompatible adhesive or other securing means. Example adhesives are thermoplastic fluoropolymers, such as fluorinated ethylene propylene (FEP). Other simple mechanical means are possible, including a compression fit collar, staples or sutures or other fastening techniques acceptable for implantation within the tissue of the body.

In one embodiment shown in FIGs. 17A and 17B, the proximal end 54 of the sleeve 44 includes an end wall 57 having a reduced axial opening 62 having a diameter smaller than the widest diameter of the tip 52. Because the material of the sleeve 44 is a semi-elastic material, the tip 52 can be forced through the opening 62, but due to the diameter of the tip 52 being larger than the opening 62, cannot be easily removed. The sleeve 44 is thus secured to the distal end of the tunneling instrument 42.

Referring now to FIG.18, in another embodiment, the distal end of the tunneling instrument 42 may be mechanically secured to the proximal end 54 of the sleeve 44 using a fastening element for securing the sleeve. The fastening element is secured directly to the tunneling instrument 42 is shown and generally designated at 70. The fastening element 70 comprises a snap-on clip that may be used to couple the sleeve 44 to the shaft 48 such that the sleeve 44 is attached to and encloses at least a portion of the distal end and the tip 52 of the tunneling instrument 42. The fastening clip comprises a C-shaped body 72 and a centrally located pin 74 in the same plane as the body 72 and extending radially inwardly into the opening defined by the body 72. The clip 70 is configured to receive and enclose a portion of the proximal end 54 of the sleeve 44. The distal end of the shaft spaced from the tip 52 defines a hole 76 configured to receive the pin 74. The proximal end 54 of the sleeve 44 is enclosed by the clip 70. The sleeve 44 may be coupled to the tip 51 by passing sleeve 44 over the tip 52 and mounting the clip 70 over the sleeve 44. This causes the arms of the body 72 to expand outwardly together until the arms clear the largest diameter of the sleeve 44 directing the pin74 into the hole 76 in the shaft through the sleeve 44 until the pin seats in the hole. The arms of the clip 70 can then spring inwardly. This fastens the sleeve 44 and tunneling instrument 42 together along with the clip 70. The user can now pull the graft 46 into the subcutaneous tunnel previously formed by the tunneling instrument 42. It is understood that the clip 70 can be used by sliding only an end portion 134, 136 of the graft 46 over the tip 52 of the tunneling instrument 42 and securing the graft with the clip 70.

In use, after the distal end of the tunneling instrument 42 without the sleeve 44 emerges from the second distal incision 82, the proximal end of the sleeve 44 is secured to the tip 52. After attachment of the sleeve 44 to the tunneling instrument 42, the sleeve 44 extends distally from the distal end of the tunneling instrument 42. The tunneling instrument 42 is extracted proximally simultaneously pulling the sleeve 44 through the previously dissected tissue defining the anatomical tunnel. Once the sleeve 44 has been drawn to the site of the first incision 80, the proximal end of the sleeve 44 is disconnected from the distal end of the tunneling instrument 42. The sleeve 44 remains in the tissue forming an internal passage through the sleeve that is sufficiently large to allow the subsequent passage of the graft 46. The graft 46 is loaded proximally, as described above, and the sleeve 44 extracted distally, which simultaneously draws the attached graft 46 into and through the tunnel with the sleeve 44 until the proximal end 54 of the graft 46 exits the second incision 82. The sleeve 44 allows the surgeon to easily pull the vascular graft 46 through the anatomical tunnel with the sleeve 44, which is substantially oversized in comparison to the outside diameter of the vascular graft 46.

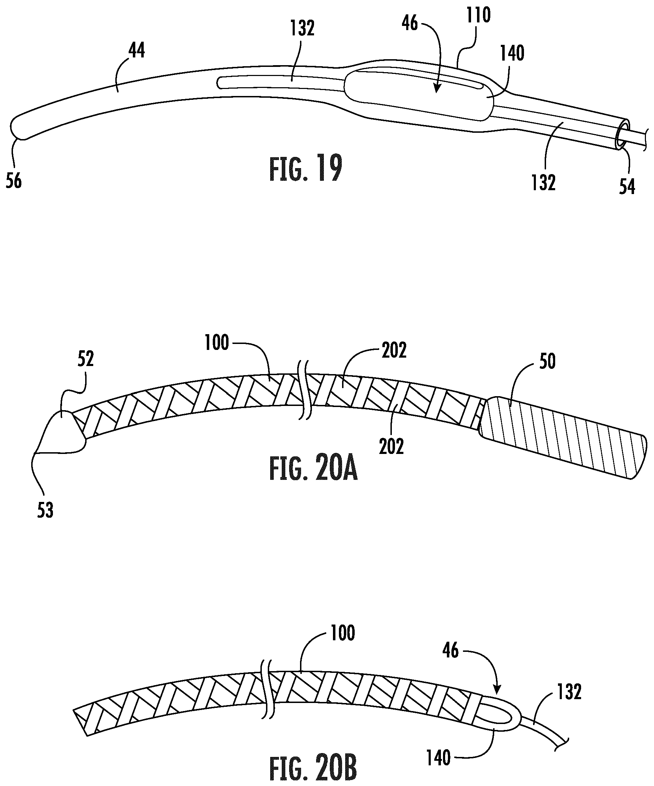

FIG. 19 shows an embodiment of the apparatus comprising the sleeve 44, which includes an enlarged, bulbous portion 110 spaced from the ends 54, 56 for receiving the cannulation chamber 40 of the graft 46. The enlarged diameter of the bulbous portion 110 of the sleeve 44 allows the sleeve 44 to receive the entire cannulation chamber 140 of the graft 46. Some force may be required to mount the graft 46 in the lumen of the sleeve 44.

FIG. 20 shows an embodiment wherein the sleeve is generally designated at 200. The sleeve 200 is woven, and each of the threads 202 of the weave can move independent of one another. The sleeve functions similarly to the previous embodiment.

The apparatus and method for implanting the arteriovenous graft, including a sleeve 44 enclosing the tunneling instrument 42 for deploying the graft 46 during implantation, have many advantages, including atraumatic implantation of an arteriovenous graft 46 and subsequent extraction of the associated sleeve 44. The sleeve 44 is a simple addition to conventional tunneling instruments. The sleeve provides a flexible, compressible yet stiff outer surface for the shaft 48 of the tunneling instrument 42 that may allow for easier tunneling with less friction and resistance, and related tissue damage, during the passage of the tunneling device when forming an anatomical tunnel. Insertion of the graft 46 with the sleeve 44 into the tissue cavity occurs with less trauma, less friction, and less drag during placement. Thus, the system and method described herein may reduce damaging forces to surrounding tissues associated with the implant procedure and minimize the resultant trauma to this tissue and its healing response. Due to the smoothness and collapsible low profile of the sleeve 44, the tunneling procedure may be faster and easier to use. The delivery system will allow the surgeon to avoid the use of sutures to attach the graft to the tunneler. This facilitates immediate or early cannulation of a subsequently implanted vascular graft.

The embodiments of the tunneling apparatus as described herein are shown in use for procedures with a vascular graft suitable for implantation in the body and used to reestablish or redirect the flow of blood beyond a blockage area. Implantation is a required surgical step in peripheral vascular procedures for all peripheral, vascular access and extra-anatomical graft locations. The arteriovenous graft is positioned in the tunnel within the bodily tissue for fixation of the graft to an existing peripheral vessel to form a bypass around the vessel, or a portion thereof, or connection of an artery and vein to form an arteriovenous shunt. The vascular graft may also connect an artery to an artery. One of ordinary skill in the art will also recognize that the embodiments of the tunneling apparatus as described are not directed to a specific vascular graft design, but are generically applicable to many different types of vascular grafts, which may be a synthetic graft constructed from different materials or a natural tissue graft. Accordingly, it is understood that the several tunneling apparatuses described and shown herein may be used with more arteriovenous grafts than those shown in the drawings, including grafts without cannulation chambers, biologic grafts and fistulas. In addition, the apparatus and method may be used in other surgical implantation procedures requiring placement of a medical device or other object within the subcutaneous tissue.

Further embodiments of the disclosure are defined in the following clauses:
Clause 1: An apparatus for subcutaneous delivery in tissue of a patient of a vascular arteriovenous graft, including a cannulation chamber and having a proximal end and a distal end and a length extending along a longitudinal axis between the proximal end and the distal end, using a rigid tunneling instrument including an elongated shaft having a handle at a proximal end of the shaft and a removable tip at a distal end of the shaft for creating a pathway in the subcutaneous tissue, the subcutaneous delivery apparatus comprising:
   an elongated tubular sleeve having a length and defining an interior lumen, the sleeve adapted for being slidably positioned over at least a portion of the shaft of the tunneling instrument between the handle and the tip such that once the tunneling instrument has been advanced to a desired subcutaneous anatomical location the shaft is selectively removed from the sleeve while the sleeve remains positioned at the desired anatomical location, the sleeve having a slit extending along the length of the sleeve from the proximal end to a point intermediate the length of the sleeve,
   wherein the lumen defined at the proximal end of the sleeve is adapted to receive the distal end of the graft and at least a portion of the cannulation chamber such that the proximal end portion of the sleeve progressively expands along the slit to provide an enlarged diameter for accommodating the cannulation chamber, and
   wherein application of a longitudinal force to the sleeve from the distal end of the sleeve is effective to cause the sleeve to move in a distal direction during removal of the sleeve from the tissue such that the graft is fixed in the sleeve by radial compression of the sleeve for pulling the graft and the sleeve through the tunnel for deploying the vascular graft.
Clause 2: The subcutaneous delivery apparatus as recited in clause 1, wherein the sleeve is adapted to receive substantially the entire length of the shaft between the handle and the tip.
Clause 3: The subcutaneous delivery apparatus as recited in clause 1, wherein the sleeve is uniformly tapered from the proximal end to the distal such that distal end has a reduced diameter from the proximal end.
Clause 4: The subcutaneous delivery apparatus as recited in clause 1, wherein the slit is linear.
Clause 5: The subcutaneous delivery apparatus as recited in clause 1, wherein the sleeve comprises an enlarged diameter portion adjacent the proximal end, the enlarged diameter portion of the sleeve adapted for receiving the cannulation chamber. Clause 6: An apparatus for subcutaneous implantation in tissue of a patient of a vascular arteriovenous graft including a cannulation chamber and having a proximal end and a distal end and a length extending along a longitudinal axis between the proximal end and the distal end, the subcutaneous implantation apparatus comprising:
   a rigid tunneling instrument for creating a pathway in the subcutaneous tissue, the tunneling instrument including
      an elongated shaft having a handle at a proximal end of the shaft, and
      a removable tip at a distal end of the shaft; and
   an elongated tubular sleeve having a length and defining an interior lumen, the sleeve having a slit extending along the length of the sleeve from the proximal end to a point intermediate the length of the sleeve, the sleeve configured for being slidably positioned over at least a portion of the shaft of the tunneling instrument between the handle and the tip,

   wherein once the tunneling instrument has been advanced to a desired subcutaneous anatomical location the shaft is selectively removed from the sleeve while the sleeve remains positioned at the anatomical location,
   wherein the lumen is adapted to receive the distal end of the graft and at least a portion of the cannulation chamber via the proximal end of the sleeve such that the proximal end portion of the sleeve progressively expands along the slit to provide an enlarged diameter for accommodating the cannulation chamber, and
   wherein application of a longitudinal force to the sleeve from the distal end of the sleeve is effective to cause the sleeve to move in a distal direction for removal of the sleeve from the tissue such that the arteriovenous graft is fixed in the sleeve by radial compression of the tissue around the sleeve for pulling the arteriovenous graft and the sleeve through the tunnel deploying the vascular graft.
Clause 7: The subcutaneous implantation apparatus as recited in clause 6, wherein the sleeve is configured to receive substantially the entire length of the shaft between the handle and the tip.
Clause 8: The subcutaneous implantation apparatus as recited in clause 6, wherein the sleeve is uniformly tapered from the proximal end to the distal such that distal end has a reduced diameter from the proximal end.
Clause 9: The subcutaneous implantation apparatus as recited in clause 6, wherein the slit is linear.
Clause 10: The subcutaneous implantation apparatus as recited in clause 6, wherein the sleeve comprises an enlarged diameter portion adjacent the proximal end, the enlarged diameter portion adapted for receiving the cannulation chamber.
Clause 11: The implantation apparatus as recited in clause 6, wherein the sleeve comprises a terminal wall at the proximal end, the wall having an axial opening for receiving the tip of the tunneling instrument for connection of the sleeve to the tunneling instrument.
Clause 12. The subcutaneous delivery apparatus as recited in clause 6, further comprising a clip including a pin, wherein the shaft has a hole adjacent the tip defining an axial passage for receiving the pin and capturing the sleeve between the clip and the shaft.
Clause 13. A system for subcutaneous delivery of a medical device in a patient, the subcutaneous delivery device comprising:
   a rigid tunneling instrument for creating a pathway in the subcutaneous tissue, the tunneling instrument including
      an elongated shaft having a handle at a proximal end of the shaft, and
      a removable tip at a distal end of the shaft;
   a vascular arteriovenous graft including a cannulation chamber and having a proximal end and a distal end and a length extending along a longitudinal axis between the proximal end and the distal end; and
   an elongated tubular sleeve having a length and defining an interior lumen, the sleeve having a slit extending along the length of the sleeve from the proximal end to a point intermediate the length of the sleeve, the sleeve configured for being slidably positioned over at least a portion of the shaft of the tunneling instrument between the handle and the tip,

   wherein once the tunneling instrument has been advanced to a desired subcutaneous anatomical location the shaft is selectively removed from the sleeve while the sleeve remains positioned at the anatomical location,
   wherein the lumen is configured to receive the distal end of the graft and at least a portion of the cannulation chamber via the proximal end of the sleeve such that the proximal end portion of the sleeve progressively expands along the slit to provide an enlarged diameter for accommodating the cannulation chamber, and
   wherein application of a longitudinal force to the sleeve from the distal end of the sleeve is effective to cause the sleeve to move in a distal direction for removal of the sleeve from the tissue such that the arteriovenous graft is fixed in the sleeve by radial compression of the tissue around the sleeve for pulling the arteriovenous graft and the sleeve through the tunnel deploying the vascular graft.
Clause 14. The subcutaneous delivery system as recited in clause 13, wherein the sleeve is configured to receive substantially the entire length of the shaft between the handle and the tip.
Clause 15. The subcutaneous delivery system as recited in clause 13, wherein the sleeve is uniformly tapered from the proximal end to the distal such that distal end has a reduced diameter from the proximal end.
Clause 16. The subcutaneous delivery system as recited in clause 13, wherein the slit is linear.
Clause 17. The subcutaneous delivery system as recited in clause 13, wherein the sleeve comprises an enlarged diameter portion adjacent the proximal end, the enlarged diameter portion configured for receiving the cannulation chamber.
Clause 18. The subcutaneous delivery system as recited in clause 13, wherein the sleeve comprises a wall at the proximal end, the wall having an axial opening for receiving the tip of the tunneling instrument for connection of the sleeve to the tunneling instrument.
Clause 19. The subcutaneous delivery system as recited in clause 13, further comprising a clip including a pin wherein the shaft has a hole adjacent the tip defining an axial passage for receiving the pin for connecting the shaft to the sleeve for applying a pulling force in a proximal direction.
Clause 20. A method for subcutaneously implanting an arteriovenous graft in tissue of a subject, the arteriovenous graft including a cannulation chamber and having a proximal end and a distal end and a length extending along a longitudinal axis between the proximal end and the distal end, the implanting method comprising the steps of:
   incising tissue of the subject in a first proximal location and second distal location spaced from the first proximal location;
   providing a rigid tunneling instrument including an elongated shaft having a handle at a proximal end of the shaft and a removable tip at a distal end of the shaft; removing the tip and disposing a tubular sleeve over at least a portion of the shaft between the tip and handle, the sleeve having a slit extending along the length of the sleeve from the proximal end to a point intermediate the length of the sleeve;
   securing the tip to the distal end of the shaft and inserting the tip into the first incision and subcutaneously advancing the instrument and the sleeve through the tissue along a path until the tip exits the second incision such that the distal end of the sleeve extends from the second incision and the proximal end of the sleeve extends from the first incision;
   removing the tip and pulling the tunneling instrument proximally by the handle for removal of the shaft from the sleeve in the tissue;
   inserting the distal end of the graft into the proximal end of the sleeve until the cannulation chamber at least partially enters the sleeve causing the sleeve to expand along the slit to accommodate at least a portion of the chamber; and pulling the sleeve distally for extracting the sleeve from the tissue while the proximal end of the sleeve is compressed by the dissected tissue defining the tunnel forcing the sleeve against the chamber for pulling the graft through the tissue with the sleeve.
Clause 21. The method as recited in clause 20, further comprising the step of pulling the proximal end of the arteriovenous graft proximally for drawing the cannulation chamber of the arteriovenous graft distally back into the tissue.

## Claims

1. An apparatus (40) for subcutaneous implantation in tissue of a patient of a vascular arteriovenous graft (48), wherein the vascular arteriovenous graft (48) includes a cannulation chamber (140) and has a proximal end and a distal end and a length extending along a longitudinal axis between the proximal end and the distal end, the subcutaneous implantation apparatus comprising:
a rigid tunneling instrument (42) for creating a pathway in the subcutaneous tissue,
the tunneling instrument (42) including
an elongated shaft (48) having a handle (50) at a proximal end of the shaft (48), and
a removable tip (52) at a distal end of the shaft (48); and
an elongated tubular sleeve (44) having a length and defining an interior lumen, the sleeve (44) having a slit (58) extending along the length of the sleeve (44) from a proximal end (54) to a point intermediate the length of the sleeve (44), the sleeve configured for being slidably positioned over at least a portion of the shaft (48) of the tunneling instrument (42) between the handle (50) and the tip (52),
wherein, the apparatus is configured such that, in use, once the tunneling instrument (42) has been advanced to a desired subcutaneous anatomical location, the shaft (50) is configured to be selectively removed from the sleeve (44) while the sleeve (44) is configured to remain positioned at the anatomical location,
wherein the lumen is adapted to receive the distal end of the graft (48) and at least a portion of the cannulation chamber (140) via the proximal end (54) of the sleeve (44) such that a proximal end portion (54) of the sleeve (44) is configured to progressively expand along the slit (58) to provide an enlarged diameter for accommodating the cannulation chamber (140), and
wherein, in use, the application of a longitudinal force to the sleeve (44) from a distal end (56) of the sleeve (44) is effective to cause the sleeve (44) to move in a distal direction for removal of the sleeve (44) from the tissue such that the arteriovenous graft (46) is fixed in the sleeve (44) by radial compression of the tissue around the sleeve (44) for pulling the arteriovenous graft (46) and the sleeve through the pathway deploying the vascular graft (46).

2. The subcutaneous implantation apparatus as recited in claim 1, wherein the sleeve (44) is configured to receive substantially the entire length of the shaft (48) between the handle (50) and the tip (52).

3. The subcutaneous implantation apparatus as recited in claim 5, wherein the sleeve (44) is uniformly tapered from the proximal end (54) to the distal end (56) such that distal end (56) has a reduced diameter from the proximal end (54).

4. The subcutaneous implantation apparatus as recited in claim 1, wherein the slit (58) is linear.

5. The subcutaneous implantation apparatus as recited in claim 1, wherein the sleeve (44) comprises an enlarged diameter portion adjacent the proximal end (54), the enlarged diameter portion adapted for receiving the cannulation chamber (140).

6. The subcutaneous implantation apparatus as recited in claim 1, wherein the sleeve (44) comprises a terminal wall (57) at the proximal end (54), the wall (57) having an axial opening (62) for receiving the tip (52) of the tunneling instrument (42) for connection of the sleeve (44) to the tunneling instrument (42).

7. The subcutaneous implantation apparatus as recited in claim 2, further comprising a clip (70) including a pin (74), wherein the shaft (48) has a hole adjacent the tip (52) defining an axial passage for receiving the pin (74) and capturing the sleeve (44) between the clip (70) and the shaft (48).

8. The subcutaneous implantation apparatus as recited in any of claims 1 to 7, further comprising:
a vascular arteriovenous graft (46) including a cannulation chamber (140) and having a proximal end and a distal end and a length extending along a longitudinal axis between the proximal end and the distal end.
